# FASCICULE DE BREVET EUROPEEN

(11) **EP 2 501 675 B1**
(45) Date de publication et mention de la délivrance du brevet: **06.12.2017**
(21) Numéro de dépôt: 10776391.4
(22) Date de dépôt: 17.11.2010
(51) Int. Cl.: C08F 12/22, C08F 12/24, C08F 12/26, C08F 12/34, C07C 251/48, C07C 251/70, C08F 212/34

(54) **COMPLEXES DE COORDINATION POLYMERISABLES ET MATERIAUX POLYMERIQUES OBTENUS A PARTIR DESDITS COMPLEXES**
POLYMERISIERBARE KOORDINATIONSKOMPLEXE UND POLYMERE ERHALTEN DURCH DIE GENANNTEN KOMPLEXE
POLYMERISABLE COORDINATION COMPLEXES AND POLYMERS OBTAINED FROM SAID COMPLEXES

(30) Priorité: 19.11.2009 FR 0958190
(43) Date de publication de la demande: 26.09.2012
(73) Titulaire: Commissariat à l'Énergie Atomique et aux Énergies Alternatives, 75015 Paris (FR)
(72) Inventeur: BALLAND LONGEAU, Alexia, F-37000 Tours (FR); CADRA, Stéphane, F-37000 Tours (FR); THIBONNET, Jérôme, F-37250 Veigne (FR)
(74) Mandataire: Ilgart, Jean-Christophe
(86) Numéro de dépôt international: PCT/EP2010/067689
(87) Numéro de publication internationale: WO 2011/061229

(56) Documents cités:
- EP-A1- 0 584 988
- US-B2- 6 749 811
- SOUTHARD GLEN E ET AL: "Heck Cross-Coupling for Synthesizing Metal-Complexing Monomers", SYNTHESIS, GEORG THIEME VERLAG, STUTTGART, DE LNKD- DOI:10.1055/S-2006-942471, no. 15, 1 août 2006 (2006-08-01), pages 2475-2477, XP009135738, ISSN: 0039-7881
- DIAZ F R ET AL: "Synthesis and characterization of polymers derived from salicylaldoxime and their corresponding chelates with copper(II) or iron(III)", BOLETIN DE LA SOCIEDAD CHILENA DE QUIMICA, SOCIEDAD CHILENA DE QUIMICA, CONCEPCION, CL, vol. 36, no. 4, 1 janvier 1991 (1991-01-01), pages 253-258, XP009135740, ISSN: 0366-1644
- DATABASE BEILSTEIN [Online] BEILSTEIN INSTITUTE FOR ORGANIC CHEMISTRY, FRANKFURT-MAIN, DE; 1903, XP002590737, Database accession no. BRN 197381 & FREUND ET AL: CHEMISCHE BERICHTE, vol. 36, 1903, page 1533, ISSN: 0002-7864

## Description

### DOMAINE TECHNIQUE

La présente invention a trait à de nouveaux complexes de coordination polymérisables comprenant des éléments métalliques, tels que le titane, le zirconium, l'hafnium, le vanadium, le niobium, le tantale, et à des matériaux polymériques résultant de la polymérisation desdits complexes.

Ces complexes de coordination et les matériaux obtenus par polymérisation de ceux-ci trouvent leur application dans le champ global des applications propres aux matériaux polymériques dopés avec des éléments métalliques, telles que la catalyse supportée, les matériaux luminescents, les matériaux magnétiques, les matériaux à empreinte ionique. En particulier, ils trouvent leur application dans l'élaboration de cibles laser utilisées lors d'expériences de fusion par confinement inertiel.

Le domaine général de l'invention est ainsi celui des matériaux polymériques dopés par un ou plusieurs éléments métalliques.

### ÉTAT DE LA TECHNIQUE ANTÉRIEURE

Eu égard au champ d'application extrêmement vaste de ce type de matériaux, de nombreuses équipes ont axé leur recherche sur des procédés d'élaboration de tels matériaux.

Une première stratégie a consisté à imprégner des matériaux polymériques par des solutions de sel métallique.

Ainsi, Rinde et al. dans US 4,261,937 décrit une méthode de préparation de mousses polymériques dopées par un élément métallique consistant à verser un gel polymérique dans une solution aqueuse comprenant un sel dudit élément métallique. Le gel est ensuite mis en présence d'une série de solvants de polarité décroissante, afin d'éliminer l'eau introduite. Chaque solvant utilisé doit être capable de solubiliser le solvant précédent et est saturé par le sel métallique choisi.

Cette méthode présente toutefois l'inconvénient majeur que la répartition de l'élément métallique ne peut pas être parfaitement homogène au niveau atomique, car il se produit des phénomènes de cristallisation de sels métalliques au séchage, qui s'ensuit par la formation de nano- ou microcristaux dans le matériau. D'autre part, du fait que l'imprégnation est réalisée sur un gel polymérique, la diffusion des éléments métalliques ne se produit pas dans la totalité du gel.

D'autres auteurs ont utilisé des variantes de ce type de stratégie.

Ainsi, Mishra et al., dans Plasma Phys. Control. Fusion 43 (2001) 1723-1732, décrit la préparation de microballons en polystyrène dopés par des particules métalliques ultrafines comprenant les étapes suivantes :
- une étape de formation d'une émulsion comprenant une phase aqueuse et une phase organique comprenant du polystyrène, dans laquelle sont dispersées des particules métalliques ultrafines ;
- une étape de dispersion de l'émulsion susmentionnée dans une seconde phase aqueuse, moyennant quoi l'on obtient une émulsion triphasique ;
- une étape d'élimination de la phase organique, laissant ainsi subsister des ballons de polystyrène dopés par des particules métalliques contenant de l'eau ;
- une étape de séchage desdits ballons de polystyrène.

Une deuxième stratégie a consisté, non plus à doper les matériaux après polymérisation de ces derniers, mais à agir en amont de l'étape de polymérisation en mettant l'élément métallique en contact avec le milieu de polymérisation, notamment par l'utilisation de monomères porteurs du métal dopant souhaité, selon que le métal fait partie intégrante de la molécule de monomère (auquel on parle de monomères métalliques) ou est lié à celle-ci par une réaction de complexation.

Ainsi, certains auteurs ont axé leurs travaux de recherche sur la synthèse de monomères vinyliques comprenant des éléments métalliques ou aptes à être dopés par des éléments métalliques, tels que le titane.

C'est le cas de Miele-Pajot et al., dans J. Mater. Chem., 1999, 9, 3027-3033, qui décrit la formation d'un complexe de titane obtenu par réaction d'un alcoxyde de titane, le tétraisopropoxyde de titane, avec le cis-but-2-ène-1,4-diol HO-CH₂-CH=CH-CH₂-OH, lequel complexe est ensuite mis en contact avec un milieu de polymérisation comprenant du styrène et du divinylbenzène, pour donner des mousses en polystyrène dopées au titane. Toutefois, le mode de réalisation décrit dans ce document présente les inconvénients suivants :
- le complexe de titane est instable en milieu aqueux, et notamment dans le milieu de polymérisation, cette instabilité pouvant se traduire par un clivage de la liaison métal-ligand ;
- le complexe de titane se dégrade ainsi, lors de la polymérisation de celui-ci avec le styrène et le divinylbenzène, générant ainsi une faible incorporation de titane dans le matériau polymérique final, le pourcentage massique en titane des matériaux polymériques ne dépassant pas 1,5%.

Il existe donc un véritable besoin pour des complexes de coordination, qui soient stables en milieu aqueux, et notamment lorsqu'ils sont mis en présence d'un milieu de polymérisation et permettant, de ce fait, l'obtention de matériaux polymériques dopés par des éléments métalliques, dont le taux de dopage est contrôlé et est, du fait de la stabilité des complexes mis en oeuvre, directement lié à leur taux d'introduction dans le milieu de polymérisation.

### EXPOSÉ DE L'INVENTION

Ainsi, l'invention a trait, selon un premier objet, à un complexe de coordination d'au moins un élément métallique choisi parmi le titane, le zirconium, le hafnium, le vanadium, le niobium, le tantale avec au moins un monomère aromatique comprenant au moins un cycle aromatique, lequel cycle comprenant au moins un groupe éthylénique, au moins un groupe hydroxyde -OH, au moins un groupe oxime et les sels de celui-ci, ledit au moins un monomère répondant à la formule (I) suivante : 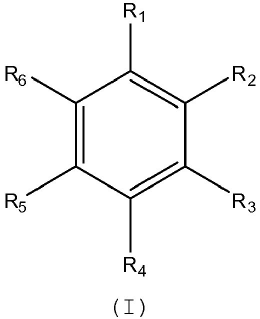 dans laquelle :
- R₁ est un groupe éthylénique ;
- R₂, R₃, R₄, R₅ et R₆ représentent, indépendamment l'un de l'autre, un atome d'hydrogène, un groupe -OH, un groupe amine, un groupe -CHO, un groupe oxime, un groupe hydrazone, un groupe carboxyle -COOH, un atome d'halogène, un groupe trialkylsilane, et les éventuels sels de ceux-ci, à condition que l'un au moins des groupes R₂ à R₆ représente un groupe -OH et l'un au moins des groupes R₂ à R₆ représente un groupe oxime ;
ledit élément métallique étant sous forme d'un alcoxyde métallique.

On précise que, par complexe de coordination, on entend classiquement un édifice polyatomique comprenant l'élément métallique autour duquel des groupes appartenant à au moins un monomère (en l'occurrence, les groupes -OH et oxime) sont liés par des liaisons de coordination, la liaison de coordination étant créée par apport d'un doublet d'électrons appartenant auxdits groupes dans une orbitale vide de l'élément métallique.

Les complexes de coordination de l'invention présentent les avantages suivants :
- ils sont stables en milieu aqueux, et notamment sous forme complexé avec des éléments métalliques, cette stabilité résidant dans la stabilité de la liaison métal-ligand des monomères sous forme complexé ;
- ils peuvent être polymérisés en vue de donner des matériaux polymériques à taux de dopage contrôlé (ce taux de dopage pouvant être très élevé), du fait de leur stabilité dans les milieux de polymérisation ;
- ils sont polymérisables, éventuellement en présence d'autres comonomères, tant en milieu organique qu'en émulsion (par exemple, un mélange d'eau et d'un ou plusieurs solvants organiques).

Les monomères entrant dans la constitution des complexes de l'invention répondent à la formule (I) suivante : 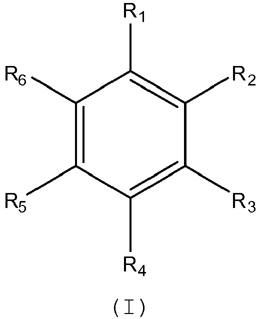 dans laquelle :
- R₁ est un groupe éthylénique ;
- R₂, R₃, R₄, R₅ et R₆ représentent, indépendamment l'un de l'autre, un atome d'hydrogène, un groupe -OH, un groupe amine, un groupe -CHO, un groupe oxime, un groupe hydrazone, un groupe carboxyle -COOH, un atome d'halogène, un groupe trialkylsilane, et les éventuels sels de ceux-ci à condition que l'un au moins des groupes R₂ à R₆ représente un groupe -OH et l'un au moins des groupes R₂ à R₆ représente un groupe oxime.

Avant d'entrer plus en détail dans la défintion des monomères de l'invention, nous précisons les définitions suivantes.

Par groupe amine, on entend, classiquement, un groupe amine primaire -NH₂, un groupe amine secondaire (à savoir, un groupe amine dont l'un des atomes d'hydrogène initialement porté par l'atome d'azote est substitué par un autre groupe, tel qu'un groupe alkyle) ou un groupe amine tertiaire (à savoir, un groupe amine dont les deux atomes d'hydrogène initialement portés par l'atome d'azote sont substitués par un autre groupe, tel qu'un groupe alkyle).

Par groupe oxime, on entend classiquement un groupe comprenant la fonction -C=N-OH, par exemple un groupe répondant à la formule -CR'=NOH, où R' représente un atome d'hydrogène, un groupe alkyle, un groupe aryle, un groupe alkylaryle, un groupe perfluoroalkyle, un groupe perfluoroaryle, un groupe perfluoroalkylaryle, un groupe acyle, un groupe carbonyle, un groupe trialkylsilane.

Par groupe hydrazone, on entend classiquement un groupe comprenant la fonction -C=N-N-, par exemple, un groupe répondant à la formule -CR'=N-NR"R"', dans laquelle R', R" et R'" représentent, indépendamment, un atome d'hydrogène, un groupe alkyle, un groupe aryle, un groupe alkylaryle, un groupe perfluoroalkyle, un groupe perfluoroaryle, un groupe perfluoroalkylaryle, un groupe acyle, un groupe carbonyle, un groupe trialkylsilane.

Par atome d'halogène, on entend, selon l'invention un atome choisi parmi le fluor, le chlore, le brome et l'iode.

Par groupe alkyle, on entend, classiquement, selon l'invention, dans ce qui précède et ce qui suit, un groupe alkyle linéaire ou ramifié comprenant de 1 à 20 atomes de carbone, un groupe cyclique comprenant de 3 à 20 atomes de carbones. On peut citer parmi ces groupes le groupe méthyle, éthyle, n-propyle, i-propyle, n-butyle, n-dodécanyle, i-butyle, t-butyle, cyclopropyle, cyclohexyle. Ces groupes peuvent comprendre dans leur chaîne un ou plusieurs atomes choisis parmi O, S, Se et/ou N.

Par groupe aryle, on entend, classiquement, selon l'invention dans ce qui précède et ce qui suit, un groupe de 6 à 20 atomes de carbone. On peut citer, parmi ces groupes, le groupe benzyle, naphtyle, tolyle, biphényle.

Par groupe alkylaryle, on entend, classiquement, selon l'invention, dans ce qui précède et ce qui suit, un groupe aryle de même définition que celle donnée précédemment, ledit groupe étant substitué par au moins une chaîne alkyle, pouvant comporter un ou plusieurs atomes d'O, de N, Se et/ou S.

Par groupe perfluoroalkyle, perfluoraryle, perfluoroalkylaryle, on entend des groupes dont les atomes d'hydrogène sont totalement substitués par des atomes de fluor (les alkyles, aryles répondant à la même définition que celle donnée précédemment). Par exemple, on peut citer le trifluorométhyle -CF₃, le perfluoroéthyle, le perfluorobutyle, le perfluoropropyle, le perfluoropentyle, le perfluorophényle C₆F₅-, le perfluorobiphényle, le perfluorobenzyle.

Comme mentionné ci-dessus, les monomères comportent au moins sur un cycle aromatique au moins un groupe -OH et au moins un groupe oxime situé sur ledit cycle aromatique.

De préférence, le groupe -OH et le groupe oxime sont situés en position *ortho* l'un de l'autre sur un même cycle aromatique, par exemple, sur un cycle phényle.

Ainsi, un monomère particulier répond à la formule (II) suivante :

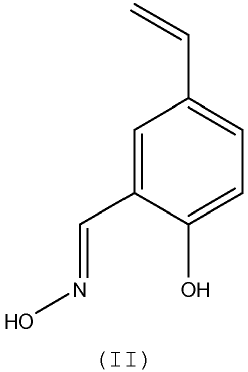

Les monomères conformes à l'invention peuvent être élaborés simplement à partir de composés de départ peu onéreux, notamment des composés naturels tels que la salicylaldéhyde.

Ainsi, en partant de la salicylaldéhyde, on peut réaliser le monomère particulier mentionné ci-dessus par les simples étapes suivantes :
- une étape d'halogénation par substitution électrophile d'un atome d'hydrogène porté par le groupe phényle de la salicylaldéhyde, cette étape pouvant consister en une étape d'iodation par action sur la salicylaldéhyde d'un sel d'iode (tel que l'iodure de chlore) en milieu acétique, moyennant quoi l'on obtient la 5-iodosalicylaldéhyde ;
- une étape de formation de la fonction oxime par réaction de l'hydroxylamine sur la 5-iodosalicylaldéhyde, moyennant quoi l'on obtient la 5-iodosalicylaldoxime ;
- une étape d'introduction du groupe éthylénique par réaction de la 5-iodosalicylaldoxime avec un composé vinylétain en présence d'un catalyseur à base de platine (tel que Pd(PPh₃)₄ avec Ph indiquant un groupe phényle).

Un tel procédé peut être résumé par le schéma réactionnel suivant :

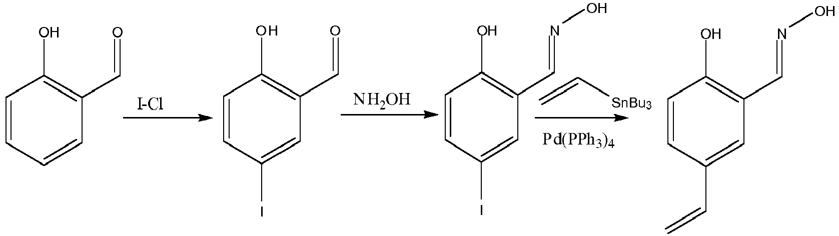

De façon générale, en partant d'un composé comprenant un cycle aromatique (par exemple, des dérivés du styrène, la salicylaldéhyde, la salicylaldoxime, le phénol), il est tout à fait à la portée de l'homme du métier, par des techniques de synthèse simples, d'introduire les groupes primordiaux des monomères de l'invention, à savoir, si ces groupes ne sont pas déjà présents dans les composés de départ, les groupes éthylénique, hydroxyle et/ou oxime.

Les monomères peuvent être fabriqués, dans des conditions douces, notamment lorsqu'il s'agit d'introduire un groupe éthylénique sur un groupe phényle halogéné, cette introduction pouvant être réalisée à pression atmosphérique avec un léger chauffage (par exemple, au maximum 50°C).

L'élément métallique susmentionné peut être un métal alcalin, un métal alcalino-terreux, un métal de transition, tel que Ti, Zr, Hf, V, Nb, Ta, un lanthanide, un actinide ainsi que les éléments Al, Ga, Ge, In, Sn, Sb, Tl, Pb, Bi ou Po, cet élément métallique pouvant se présenter d'un alcoxyde métallique.

A titre d'exemples, on peut citer les alcoxydes de titane, tels que l'isopropoxyde de titane, l'éthoxyde de titane, les alcoxydes de zirconium, tels que le n-butoxyde de zirconium, les alcoxydes de niobium, tels que l'éthoxyde de niobium.

Les complexes de coordination susmentionnés peuvent être obtenus par mise en contact des monomères tels que définis ci-dessus, éventuellement sous forme de sels, avec un alcoxyde métallique.

La réaction de formation des complexes peut être effectuée en milieu organique hydraté ou anhydre, en présence d'une atmosphère ambiante ou saturée en azote ou argon.

Des complexes de coordination spécifiques conformes à l'invention peuvent se présenter sous forme de clusters comprenant une ou plusieurs molécules de monomères entourant un ou plusieurs éléments métalliques sous forme d'alcoxydes métalliques.

A titre d'exemples, on peut citer des clusters répondant aux formules (III) et (IV) suivantes : 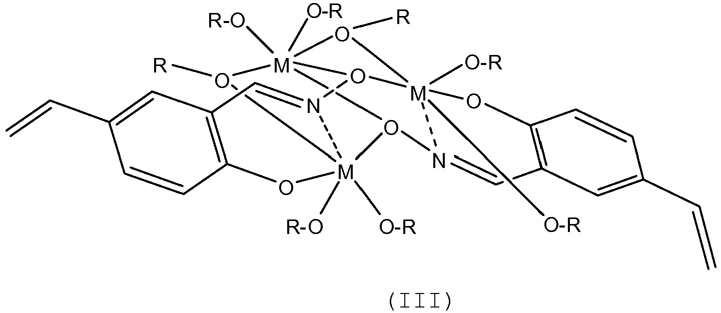 avec M représentant un élément métallique, tel que Ti, et R représentant un groupe alkyle, tel qu'un groupe isopropyle, un groupe éthyle ; 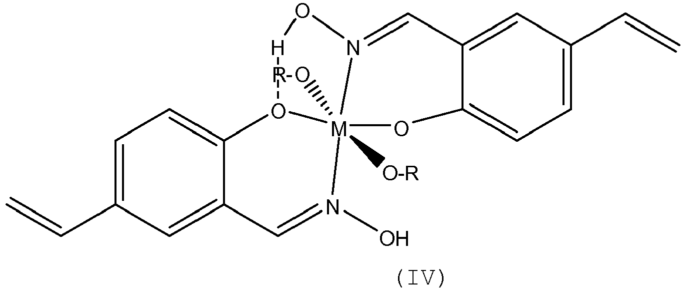 avec M représentant un élément métallique, tel que Zr, Nb et R représentant un groupe alkyle, tel qu'un groupe n-butyle, un groupe éthyle.

Les complexes de l'invention sont destinés à être utilisés pour la réalisation de matériaux polymériques dopés par au moins un élément métallique.

Ainsi, l'invention a trait, selon un troisième objet, à un procédé de préparation d'un matériau polymérique dopé par au moins un élément métallique comprenant une étape de polymérisation d'au moins un complexe de coordination tel que défini ci-dessus.

Le procédé de l'invention présente ainsi les avantages suivants :
- il permet l'incorporation, dans des matériaux polymériques, d'une large diversité d'éléments métalliques, du fait que la liaison entre les éléments métalliques et le ou les monomères s'effectue par liaison de coordination ;
- il permet une répartition de l'élément métallique à l'échelle atomique ;
- il permet une incorporation de taux élevés d'élément métallique, ledit taux étant fonction de la quantité de complexe de coordination mis en jeu lors de l'étape de polymérisation.

Classiquement, l'étape de polymérisation du procédé de l'invention se déroule, outre la présence du complexe de coordination, en présence éventuellement d'un initiateur de polymérisation et éventuellement d'un solvant porogène et d'un ou plusieurs comonomères.

Le mode de polymérisation peut être de tout type, tel que la polymérisation thermique (par exemple, en chauffant de 50 à 150°C), tel que la polymérisation photochimique en présence d'ultra-violet.

L'initiateur de polymérisation peut être un amorceur radicalaire classiquement choisi parmi les composés peroxydes, azonitriles (tel que le 2,2'-azobisisobutyronitrile), azoesters, azoamides.

L'initiateur peut être introduit, dans le milieu de polymérisation, selon des quantités variables, par exemple, selon des quantités pouvant aller de 0 à 50% massique, par rapport à la masse totale de monomères mis en jeu.

Le solvant porogène peut être un solvant organique polaire, apolaire et peut être choisi parmi les solvants éthers (tels que le tétrahydrofurane), le diméthylsulfoxyde, des solvants phthalates (tels que le diméthylphthalate, le dibutylphthalate), des solvants alcooliques (tels que le méthanol, l'éthanol), des solvants aromatiques (tels que le toluène, le fluorobenzène), des solvants cétones.

L'étape de polymérisation peut être réalisée en présence d'un ou plusieurs comonomères, lesdits comonomères étant, généralement différent des monomères entrant dans la constitution des complexes de coordination.

Ces comonomères peuvent être choisis parmi les monomères styréniques ou les monomères acrylates.

Avantageusement, les comonomères comprennent au moins deux groupes éthyléniques, assurant ainsi un rôle d'agent de réticulation. Les matériaux ainsi obtenus présentent une bonne tenue mécanique.

Des comonomères susceptibles d'être utilisés peuvent être des monomères styréniques de formule (V) suivante : 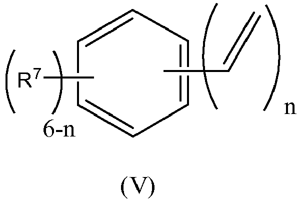 dans laquelle les (6-n) R⁷, identiques ou différents, représentent un atome d'hydrogène, un groupe alkyle, un groupe aryle, un groupe -O-aryle, un groupe -O-alkyle, un groupe acyle, un groupe alkylaryle, un atome d'halogène, lesdits groupes alkyle, aryle, alkylaryle, -O-aryle, -O-alkyle étant éventuellement perfluorés et n est un entier allant de 1 à 3, de préférence, n étant égal à 2.

En particulier, un comonomère approprié peut être le divinylbenzène, en particulier le 1,4-divinylbenzène.

Des comonomères susceptibles d'être utilisés peuvent être également des composés acrylates de formule (VI) suivante : 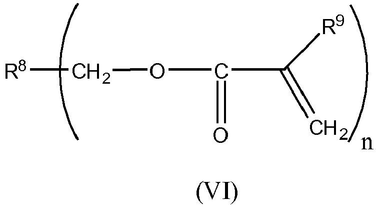 dans laquelle R⁸ représente un groupe alkyle, R⁹ représente H ou un groupe alkyle et n étant un entier allant de 1 à 3.

En particulier, un comonomère approprié de ce type peut être le triméthylolpropanetriacrylate (connu sous l'abréviation TMPTA) de formule suivante :

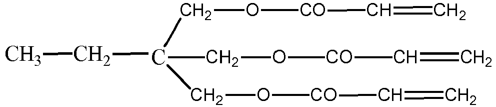

Classiquement, l'étape de polymérisation est réalisée à une température allant de 40 à 100°C.

Selon un mode particulier de l'invention, l'étape de polymérisation consiste en la copolymérisation d'un complexe de coordination de formule suivante : 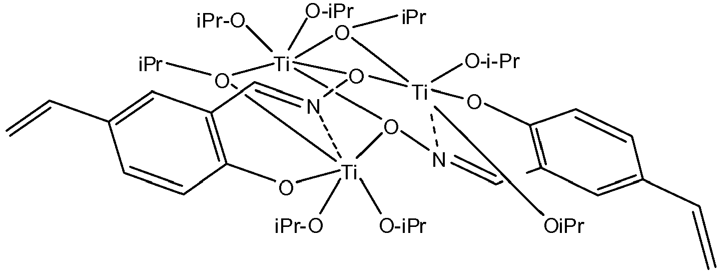 avec du divinylbenzène, iPr signifiant isopropyle ou encore avec du triméthylolpropanetriacrylate (connu sous l'abréviation TMPTA).

Après l'étape de polymérisation, un gel est obtenu, correspondant à un réseau tridimensionnel, dont la structure est imprégnée par le solvant. Le gel, une fois synthétisé, doit être séché, afin d'obtenir le matériau polymérique dopé sec.

Ainsi, le procédé comprend avantageusement une étape de séchage du gel obtenu, cette étape étant avantageusement une étape de séchage supercritique au CO₂. Pour ce faire, cette étape de séchage supercritique au CO₂ peut être précédée d'une étape d'échange de solvant consistant à remplacer le solvant présent dans les pores du gel par un solvant miscible au CO₂. Cette étape de séchage supercritique au CO₂ permet notamment de respecter l'intégrité physique de la mousse.

L'on obtient, grâce au procédé de l'invention, des matériaux polymériques dopés par un élément métallique, présentant un pourcentage élevé d'élément métallique (pouvant être supérieur à 20% massique) et avec une répartition à l'échelle moléculaire de l'élément métallique au sein du matériau.

Ainsi, l'invention a trait à des matériaux polymériques dopés par au moins un élément métallique susceptibles d'être obtenus par un procédé tel que défini ci-dessus, les matériaux se présentant classiquement sous forme de mousses.

Ces matériaux peuvent être caractérisés par une masse volumique allant de 3 à 250.10⁻³ g.cm⁻³ et une surface spécifique pouvant aller jusqu'à 880 m²/g.

Ces matériaux peuvent être utilisés dans de nombreux domaines nécessitant la mise en oeuvre de matériaux dopés par des éléments métalliques et notamment dans l'élaboration d'éléments de cibles laser utilisés, en particulier, dans des expériences de fusion par confinement inertiel.

Ils peuvent être aussi utilisés en tant que catalyseur, en tant que matériaux luminescents ou en tant que matériaux magnétiques.

En particulier, ils peuvent être utilisés en tant qu'élément de cible laser.

Enfin, ils peuvent être utilisés en tant que matériaux à empreinte ionique. Pour ce faire, les matériaux dopés obtenus par le procédé de l'invention peuvent être soumis à un traitement acide, destiné à éliminer une partie des éléments métalliques complexés dans ledit matériau. Les sites vacants constituent ainsi des empreintes spécifiques de l'élément spécifique du métal initialement introduit. De ce traitement, il en résulte un matériau dit « à empreinte ionique », capable de piéger sélectivement l'élément métallique « imprimé » lors d'une mise en contact avec un fluide comprenant ledit élément métallique. Ce type de matériaux peut ainsi être utilisé pour l'extraction sélective de métaux, notamment, lors du retraitement d'effluents de combustibles nucléaires, tel que la séparation des lanthanides, ou encore la décontamination de fluides biologiques.

L'invention va, à présent, être décrite en référence aux exemples suivants donnés à titre illustratif et non limitatif.

### EXPOSÉ DÉTAILLÉ DE MODES DE RÉALISATION PARTICULIERS

### EXEMPLE 1

Le présent exemple illustre la préparation d'un monomère de formule (II) suivante :

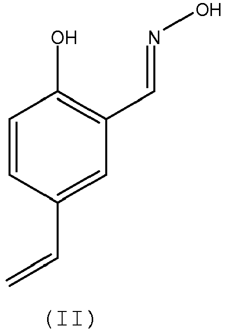

Ce monomère est réalisé selon le schéma de synthèse suivant : 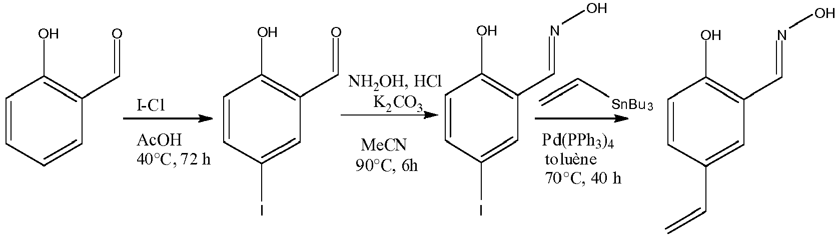 AcOH signifiant acide acétique, MeCN signifiant acétonitrile, Ph signifiant phényle, et Bu signifiant n-butyle.

Ainsi la première étape consiste à réaliser de la 5-iodosalicylaldéhyde à partir de la salicylaldéhyde.

Dans un bicol de 500 mL muni d'un réfrigérant sont placés sous agitation 160 mL d'acide acétique glacial et 10,4 mL (100 mmol) de salicylaldéhyde. Une solution de 10 g (1,1 éq., 110 mmol) de monochlorure d'iode dissous dans un minimum d'acide acétique est ensuite ajoutée au mélange. La réaction est maintenue sous agitation à T=40°C pendant 72 heures. Le solvant est ensuite évaporé. Le résidu est repris dans 100 mL de dichlorométhane, lavé avec 100 mL d'une solution aqueuse saturée en thiosulfate de sodium, 100 mL d'une solution saturée de chlorure de sodium NaCl et 150 mL d'eau distillée. La phase organique est séchée sur MgSO₄, évaporée et le résidu est recristallisé pendant 24 heures dans un minimum de dichlorométhane.

Les caractéristiques du produit obtenu sont les suivantes :
Aspect: poudre jaune
Rendement: 50%
Point de fusion: 104 °C.
RMN ¹H (solvant CPCl₃) δ : 6, 80 (lH, d, j=10Hz); 7,72 (lH, dd, j=2, 5Hz/10Hz) ; 7,84 (lH, d, j=2,5Hz); 9,82 (lH, s); 10,70 (lH, s)
RMN¹³C (solvant CDCl₃) δ : 80,81; 120, 60; 122,96; 142,26; 145,69; 161,60; 195,82.

La deuxième étape consiste à réaliser de la 5-iodosalicylaldoxime à partir de la 5-iodosalicylaldéhyde préparée préalablement.

Pour ce faire, 35 mmol (3,5 éq., 2,40 g) de chlorhydrate d'hydroxylamine sont ajoutés à une solution de 4 g de K₂CO₃ (35 mmol, 3,5 éq.) et de 5-iodosalicylaldéhyde (10 mmol, 2,50 g) dans 50 mL d'acétonitrile. Le mélange est chauffé à 70°C sous agitation pendant une nuit.

En fin de réaction, l'excès de K₂CO₃ est retiré par filtration, le filtrat est évaporé, le résidu est repris dans 250 mL d'eau, puis de l'acide chlorhydrique est introduit délicatement jusqu'à atteindre un pH de 4. La solution est ensuite extraite au dichlorométhane. La phase organique est séchée sur MgSO₄, filtrée puis le solvant est évaporé. Le solide est ensuite purifié par filtration sur silice (éluant : Heptane/Et₂O 75/25) puis le filtrat est évaporé.

Les caractéristiques du produit obtenu sont les suivantes :
Aspect: poudre blanche
Rendement: 95%
Point de fusion: 134 °C
RMN¹H (solvant CDCl₃) δ : 6,80 (lH, d,j=8Hz); 7,48 (lH, d,j=2Hz); 7,55 (1H, dd, j=2Hz/8Hz) ; 7,60 (H, s); 8,16 (lH, s); 9,85 (lH, s).
RMN¹³C (solvant CDCl₃) δ : 80,1; 117,8; 118,2; 138,2; 139,1; 151,2; 156,2.

La troisième étape consiste enfin à réaliser de la 5-vinylsalicylaldoxime à partir de la 5-iodosalicylaldoxime préparée préalablement.

Pour ce faire, dans un ballon de 100 mL, séché dans les conditions de Grignard et purgé à l'argon sont introduits, successivement, sous agitation et dans l'ordre 3,94 g (15 mmol) de 5-iodosalicylaldoxime, 30 mL de toluène anhydre, 879 mg (5% mol) de Pd(PPh₃)₄ et 6,60 mL de vinylétain CH₂=CH-SnBu₃ (Bu signifiant n-butyle). Le mélange est dégazé, purgé à l'argon et chauffé à 70°C sous forte agitation pendant 72 heures.

En fin de réaction, la solution est filtrée sur célite (avec le solvant Et₂O), le précipité se formant est éliminé par filtration, les solvants sont évaporés et le résidu est purifié par colonne flash (éluants : 400 mL d'heptane, 500 mL d'un mélange heptane/Et₂O 95 :5 et heptane/Et₂O 90 :10).

Les caractéristiques du produit obtenu sont les suivantes :
Aspect: cristaux blancs
Rendement: 51%
Point de fusion: 107 °C
RMN¹H (solvant CDCl₃) δ : 5,16 (lH, d, j=14Hz); 5,60 (lH, d, j=22,5Hz); 6,64 (1H, dd, j=13Hz/22,5Hz); 6,96 (lH, d, j=8Hz) ; 7,21 (lH, d, j=2Hz) ; 7,39 (lH, dd, j=8Hz/2Hz) ; 7,59 (lH, s); 8,24 (lH, s); 9,95 (1H, s)
RMN¹³C (solvant CDCl₃) δ : 111,78; 115,77; 116,51; 128,29; 128,55; 129,30; 135,04; 152,53; 156,42.

### EXEMPLE 2

Cet exemple a trait à la préparation d'un complexe formé par deux molécules du monomère préparé selon l'exemple 1 avec du titane sous forme d'alcoxyde (l'isopropoxyde de titane) : la 5-vinylsalicylaldoximatotitane d'isopropoxyde de formule suivante :

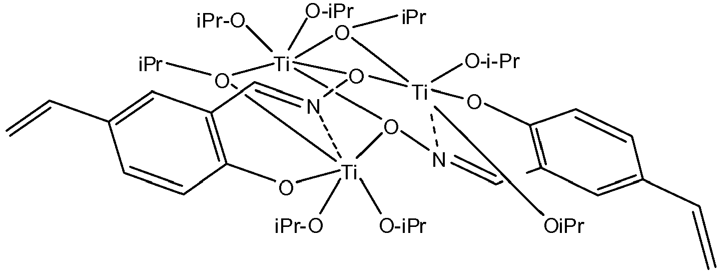

iPr signifiant isopropyle.

Dans un monocol de 15 mL est introduit 1,25 g (7,5 mmol) de 5-vinylsalicylaldoxime, 8 mL de toluène anhydre et 3,25 g (1,5 éq ; 11,25 mmol) d'isopropoxyde de titane. Le mélange est purgé à l'argon et chauffé à 50°C sous forte agitation pendant 3 heures. En fin de réaction, le solvant est évaporé. Afin d'éliminer toute trace de toluène résiduel, le résidu rouge est dilué dans 5 mL d'éther diéthylique et est à nouveau évaporé.

Les caractéristiques du produit obtenu sont les suivantes :
Aspect: solide orangé
Rendement: 93%
Point de fusion: 128,8 °C
RMN¹H (solvant toluène D8) : δ 0,99 (12H, m); 1,52 (36H, m); 4,60 (2H, sept); 4,95 (2H, sept.); 5,04 (2H, sept.); 5,18 (2H, sept.); 5,40 (2H, d, j=22Hz); 6,50 (2H, dd, j=12, 5Hz/22Hz) ; 6,80 (2H, d, j=12,5Hz); 6,98 (2H, d, j=8Hz) ; 7,01 (2H, d, j=2Hz) ; 7,20 (2H, dd, j=8Hz/2Hz); 8,15 (20H, s)
RMN¹³C (solvant toluène D8): δ 24,60; 78,15; 109,98; 117,86; 119,86; 127,93; 129,34; 135,62; 136,64; 152,93; 170,12
Analyse élémentaire: %massique Ti: 15,8 ±0.7% (15,3% théorique)
Spectrométrie de Masse Haute Résolution (HRMS): 939,3462 (939,3456 théorique); erreur +0.6 ppm

### EXEMPLE 3

Cet exemple a trait à la préparation d'un complexe formé par deux molécules du monomère préparé selon l'exemple 1 avec du titane sous forme d'alcoxyde (l'éthoxyde de titane) : la 5-vinylsalicylaldoximatotitane éthoxide de formule suivante :

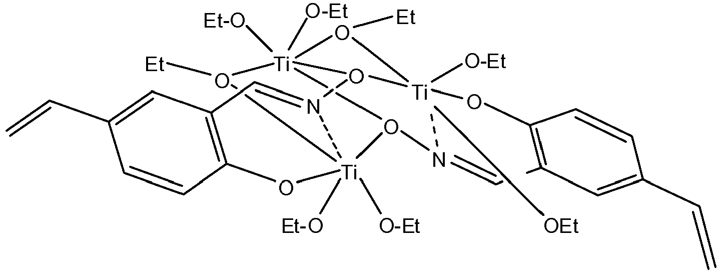

Dans un monocol de 5 mL est introduit 328 mg (2 mmol) de 5-vinylsalicylaldoxime, 2 mL de toluène anhydre et 0,629 mL (1,5 éq ; 3 mmol) d'éthoxyde de titane. Le mélange est purgé à l'argon et agité pendant 3 heures à température ambiante. En fin de réaction, le solvant est évaporé. Afin d'éliminer toute trace de toluène résiduel, le résidu rouge est dilué dans 5 mL d'éther diéthylique et est à nouveau évaporé.

Les caractéristiques du produit obtenu sont les suivantes :
Aspect: solide orangé
Rendement: 75%
RMN¹H (solvant toluène D8): δ 0,84 (6H, m); 1,26 (6H, m); 1,57 (12H, m); 4,15 (4H, quad.); 4,55 (8H, m); 4,85 (4H, quad.); 4,95 (4H, d); 5,40 (2H, d, j=22Hz); 6,50 (2H, dd, j=12,5Hz/22Hz); 6,80 (2H, d, j=12,5Hz); 6,98 (2H, d, j=8Hz); 7,01 (2H, d, j=2Hz); 7,20 (2H, dd, j=8Hz/2Hz); 8,15 (20H, s)
RMN¹³C (solvant toluène D8): δ 18,45; 71,56; 104,91; 115,80; 119,71; 126,39; 128,03; 135,48; 136,64; 157,57; 162,06

### EXEMPLE 4

Cet exemple a trait à la préparation d'un complexe formé par deux molécules du monomère préparé selon l'exemple 1 avec du zirconium sous forme d'alcoxyde (le n-butoxyde de zirconium) : la 5-vinylsalicylaldoximatozirconium n-butoxide de formule suivante :

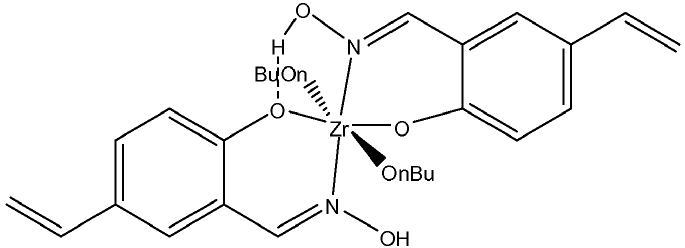

nBu signifiant n-butyle.

Dans un monocol de 5 mL purgé à l'argon est introduit 164 mg (1 mmol) de 5-vinylsalicylaldoxime solubilisé dans 2 mL de n-butanol anhydre et 0,5 mL d'une solution de n-butoxyde de zirconium à 80% massique dans le n-butanol. Le mélange est purgé à l'argon et est agité vigoureusement 2 heures à température ambiante.

En fin de réaction, le précipité jaune obtenu est isolé par filtration et rincé avec 2*10 mL de n-butanol.

Les caractéristiques du produit obtenu sont les suivantes :
Aspect: solide jaune
Rendement: 41%
RMN¹H (solvant DMSO D6) : δ 0,86 (12H, t); 1,35 (16H, m); 3,37 (4H, t); 5,11 (2H, d); 5,62 (2H, d); 6,63 (2H, dd) ; 6, 85 (2H, d) ; 7, 34 (2H, dd) ; 7, 58 (2H, d) ; 8, 32 (2H, s); 10,22 (lH, s); 11,38 (lH, s)
RMN¹³C (solvent DMSO D6): δ 19,47; 24,26; 40,30; 66,00; 117,42; 121,89; 123,90; 131,29; 133,77; 134,33; 141,54; 152,87; 161,47

### EXEMPLE 5

Cet exemple a trait à la préparation d'un complexe formé par deux molécules du monomère selon l'exemple 1 avec du niobium sous forme d'alcoxyde (l'éthoxyde de niobium): la 5-vinylsalicylaldoximatoniobium(V) éthoxide de formule suivante :

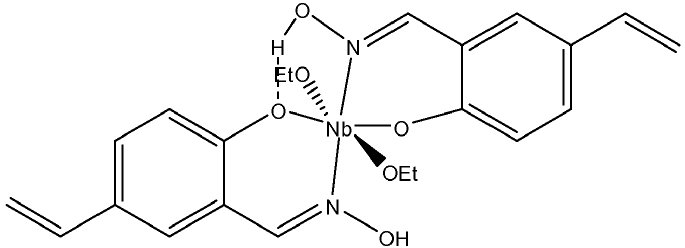

Et signifiant éthyle.

Dans un monocol de 5 mL purgé à l'argon sont introduits 164 mg (1 mmol) de 5-vinylsalicylaldoxime solubilisés dans 1,5 mL d'éthanol absolu.

Le mélange est dégazé, puis 160 mg de Nb(OEt)₅ sont introduits. Le mélange est purgé à l'argon et est agité vigoureusement une nuit à température ambiante.

En fin de réaction, le solvant est évaporé. Le solide rouge résiduel est ensuite conservé sous atmosphère inerte (N₂).
Aspect: solide rouge
Rendement: 43%
RMN¹H (solvant DMSO D6) : δ 1,05 (6H, t); 3,43 (4H, m); 5, 09 (2H, d) ; 5, 64 (2H, d) ; 6, 65 (2H, dd) ; 6, 86 (2H, d) ; 7, 37 (2H, dd) ; 7, 57 (2H, d) ; 8, 31 (2H, s); 10,20 (lH, s); 11,35 (lH, s)
RMN¹³C (solvant DMSO D6) : δ 19,16; 56,74; 112,37; 117,02; 119,07; 126,32; 128,74; 129,60; 136,55; 147,72; 156,27

### EXEMPLE 6

Cet exemple illustre la préparation d'un matériau polymérique obtenu par copolymérisation d'un complexe préparé selon l'exemple 2 avec du divinylbenzène en présence d'un solvant porogène : le dibutylphtalate (DBP).

Le mélange de monomères est préparé selon les deux méthodes suivantes.

### Méthode A

Dans un pilulier de 25 mL muni d'un agitateur et purgé à l'argon est introduit 1 g d'un mélange comprenant le complexe préparé selon l'exemple 2 et du divinylbenzène, 10 mL de dibutylphtalate, 100 mg d'azoisobutyronitrile et 100 mg de tensioactif SPAN 80. Le mélange est placé sous agitation et subit un dégazage à l'argon pendant 10 minutes.

### Méthode B

Deux solutions mères sont préparées dans deux piluliers de 25 mL :
- la première est composée de 1 g de divinylbenzène, 10 mL de dibutylphtalate, 100 mg d'azoisobutyronitrile et 100 mg de tensioactif SPAN 80 ;
- la deuxième est composée de 1 g du complexe préparé selon l'exemple 2, 10 mL de dibutylphtalate, 100 mg d'azoisobutyronitrile et 100 mg de tensioactif SPAN 80.

Les prélèvements des deux solutions sont combinés dans un pilulier de 4 mL muni d'un agitateur selon la concentration en monomère dopé désirée. La solution fille ainsi obtenue est homogénéisée et subit un dégazage à l'argon pendant 3-5 minutes.

La solution de monomères préparée selon la méthode B est introduite dans une série de moules en polypropylène (ou en silicone) préalablement purgés à l'argon. L'ensemble est placé une nuit dans une étuve à 80°C. Les gels de polymère ainsi obtenus sont ensuite démoulés et placés dans des piluliers de 25 mL contenant 15 mL d'éthanol. L'éthanol des piluliers est renouvelé toutes les 48 heures durant une semaine. Cette méthode est dénommée ci-après « méthode de polymérisation en masse ».

La solution de monomères préparée préalablement selon la méthode A est introduite au goutte-à-goutte via une seringue dans un récipient de 1 L contenant 300 mL d'une solution d'alcool polyvinylique à 88% à 50 g/L et préchauffée à 45°C, de sorte à former une multitude de billes de phase organique à l'intérieur de la phase aqueuse. Le récipient est ensuite placé à l'horizontale, en légère rotation dans un bain marie (à l'évaporateur rotatif) à 85°C pendant trois heures. Les billes de polymère ainsi obtenues sont récupérées, placées dans un pilulier de 25 mL contenant 15 mL d'eau distillée, puis rincées avec 2*15 mL d'eau distillée et 1*15 mL d'éthanol. Les billes sont conservées dans l'éthanol, le solvant étant renouvelé toutes les 48 heures pendant une semaine. Cette méthode est dénommée ci-après « méthode de polymère en émulsion ».

Les gels ainsi obtenus par ces deux méthodes de polymérisation sont ensuite séchés au dioxyde de carbone supercritique, de manière à obtenir des mousses en copolymère.

Différents essais ont été menés avec différents ratios (Complexe de l'exemple 2/DVB), à savoir des ratios massiques en complexe de 50%, 66%, 75% et 100%, moyennant quoi l'on obtient les pourcentages massiques d'élément titane suivants : 7,65%, 10,1%, 11,47% et 15,3%, les pourcentages précédents correspondant aux pourcentages en masse de titane par rapport à la masse totale de monomères de la solution préparée préalablement (appelé par la suite % massique initial de titane).

Les mousses obtenues, pour les différents ratios susmentionnés, ont fait l'objet des mesures suivantes :
- mesure du pourcentage massique de titane dans les mousses (cette mesure étant effectuée par microanalyse);
- mesure de la surface spécifique par la méthode BET pour les mousses obtenues par la méthode de polymérisation en masse.

Les résultats sont regroupés dans le tableau ci-dessous.

| | | | | |
|---|---|---|---|---|
| % initial massique de Ti | 7, 65 | 10,1 | 11,47 | 15,3 |
| % massique de Ti dans la mousse (méthode de polymérisation en masse) | 7, 9 ±0,3 | 10,4± 0,4 | 12,5± 0,5 | 17,5±0,7 |
| % massique de Ti dans la mousse (méthode de polymérisation en émulsion) | 11,3 ±0,5 | 17,2±0,7 | 19,3±0,8 | - |
| Surface spécifique (en m²/g) des mousses obtenues par la méthode de polymérisation en masse | 680±60 | 500±50 | - | 345±30 |

Il ressort de ce tableau une teneur très importante de titane dans les mousses obtenues ainsi que des valeurs de surface spécifique très importantes.

### EXEMPLE 7

Cet exemple illustre la préparation d'un matériau polymérique obtenu par copolymérisation d'un complexe préparé selon l'exemple 2 avec du triméthylolpropanetriacrylate en présence d'un solvant porogène : le dibutylphtalate (DBP).

Le mélange de monomères est préparé selon la méthode suivante.

Dans un pilulier de 25 mL muni d'un agitateur et purgé à l'argon sont introduits 1 g de mélange comprenant le complexe selon l'exemple 2 et du triméthylolpropanetriacrylate, 10 mL de DBP, 100 mg (10% massique) de tensioactif (SPAN 80) et 100 mg (10% massique) d'AIBN. Le mélange est placé sous agitation et subit un dégazage à l'argon pendant 10 minutes.

Le mélange de monomères préparé ci-dessus est polymérisé selon un procédé de polymérisation en suspension dans une phase continue aqueuse comme explicité ci-dessous.

*Préparation de la phase continue :* Dans une bouteille en verre de 5 L munie d'un agitateur magnétique sont introduits 2 L d'eau distillée. 105 g d'alcool polyvinylique (85% hydrolysé) sont ensuite ajoutés sous une agitation vigoureuse. Le mélange est chauffé à 60 °C sous agitation jusqu'à ce que la solution soit parfaitement limpide.

*Polymérisation :* 400 mL de phase continue sont prélevés et introduits dans un récipient cylindrique en verre (bouteille) de 2 L muni d'un rodage. La solution est préchauffée à 45 °C. 1 mL de mélange de polymérisation (préparé selon la *méthode A*) est ensuite introduit dans une seringue munie d'une aiguille de dimension 0,6x25 cm. La phase de monomères est introduite goutte à goutte, aiguille immergée à l'intérieur de la phase continue, de manière à former des billes de phase organique dispersées dans la phase aqueuse. Le récipient est ensuite fixé horizontalement sur un axe motorisé (par exemple une canule d'évaporateur rotatif). Celui-ci est placé en rotation lente dans un bain marie à 85 °C pendant 3 h. En fin de polymérisation, le bain-marie est retiré et la rotation est maintenue jusqu'à ce que le milieu refroidisse à une température de ∼45 °C. Les billes de gel sont prélevées et lavées à 3 reprises avec 20 mL d'eau distillée. Elles sont ensuite conservées pendant une semaine dans des piluliers de 25 mL contenant 20 mL d'éthanol absolu, cette solution d'éthanol étant renouvelée 3 fois au cours de cette période. Les gels sont ensuite récupérés et séchés par CO₂ supercritique afin d'obtenir des billes d'aérogels organiques.

Différents essais ont été menés avec différents ratios (Complexe de l'exemple 2/TMPTA), à savoir des ratios massiques en complexe de 25%, 75%, moyennant quoi l'on obtient les pourcentages massiques d'élément titane respectifs suivants : 6,5% et 19,5% (soit respectivement en % atomique, 1,1 et 3,8% atomique).

## Revendications

1. Complexe de coordination d'au moins un élément métallique choisi parmi le titane, le zirconium, le hafnium, le vanadium, le niobium, le tantale avec au moins un monomère répondant à la formule (I) suivante : 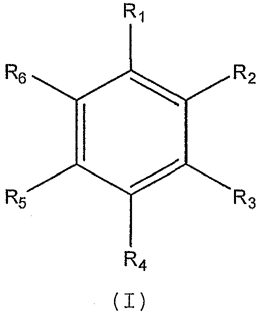 dans laquelle :
- R₁ est un groupe éthylénique ;
- R₂, R₃, R₄, R₅ et R₆ représentent, indépendamment l'un de l'autre, un atome d'hydrogène, un groupe -OH, un groupe amine, un groupe -CHO, un groupe oxime, un groupe hydrazone, un groupe carboxyle -COOH, un atome d'halogène, un groupe trialkylsilane, et les éventuels sels de ceux-ci, à condition que l'un au moins des groupes R₂ à R₆ représente un groupe -OH et l'un au moins des groupes R₂ à R₆ représente un groupe oxime ;
ledit élément métallique étant sous forme d'un alcoxyde métallique.

2. Complexe selon la revendication 1, dans lequel le monomère comprend au moins un groupe -OH et au moins un groupe oxime situés en position ortho l'un de l'autre sur un même cycle aromatique.

3. Complexe selon l'une quelconque des revendications précédentes, dans lequel le monomère répond à la formule (II) suivante :

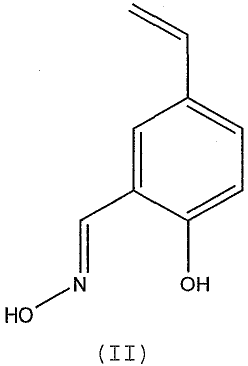

4. Complexe selon l'une quelconque des revendications précédentes, dans lequel l'élément métallique est le titane.

5. Complexe selon l'une quelconque des revendications précédentes, répondant à l'une des formules (III) et (IV) suivantes : 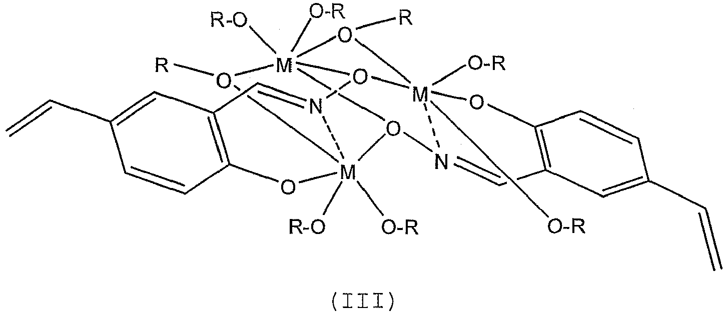 avec M représentant un élément métallique et R représentant un groupe alkyle ; 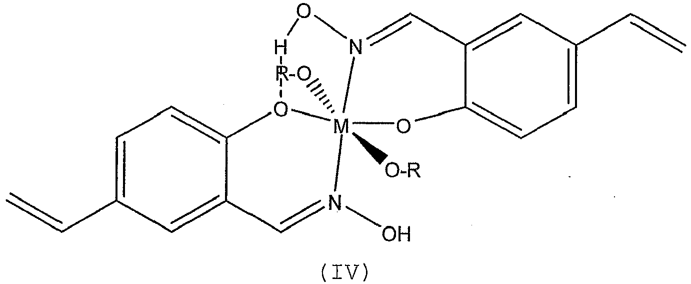 avec M représentant un élément métallique et R représentant un groupe alkyle.

6. Procédé de préparation d'un matériau polymérique dopé par au moins un élément métallique comprenant une étape de polymérisation d'au moins un complexe de coordination tel que défini selon l'une quelconque des revendications 1 à 5.

7. Procédé selon la revendication 6, dans lequel l'étape de polymérisation est réalisée en présence d'un ou plusieurs comonomères.

8. Procédé selon la revendication 7, dans lequel le ou les comonomères sont choisis parmi les monomères styréniques et les monomères acrylates.

9. Procédé selon la revendication 7 ou 8, dans lequel le ou les comonomères comprennent au moins deux groupes éthyléniques.

10. Procédé selon la revendication 8 ou 9, dans le ou les comonomères répondent à l'une des formules (V) ou (VI) suivantes : 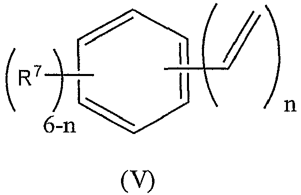 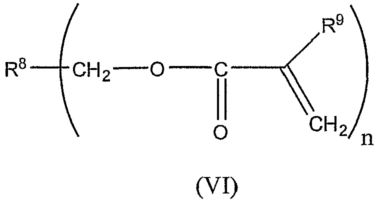 dans lesquelles les (6-n) R⁷, identiques ou différents, représentent un atome d'hydrogène, un groupe alkyle, un groupe aryle, un groupe -O-aryle, un groupe -O-alkyle, un groupe acyle, un groupe alkylaryle, un atome d'halogène, lesdits groupes alkyle, aryle, alkylaryle, -O-aryle, -O-alkyle étant éventuellement perfluorés, R⁸ représente un groupe alkyle, R⁹ représente H ou un groupe alkyle et n étant un entier allant de 1 à 3.

11. Procédé selon la revendication 10, dans lequel le comonomère est le divinylbenzène.

12. Procédé selon la revendication 10, dans lequel le comonomère est le triméthylolpropanetriacrylate.

13. Procédé selon l'une quelconque des revendications 6 à 12, comprenant, après l'étape de polymérisation, une étape de séchage supercritique au CO₂.

14. Procédé selon l'une quelconque des revendications 6 à 13, dans lequel l'étape de polymérisation consiste en la copolymérisation d'un complexe de coordination de formule suivante : 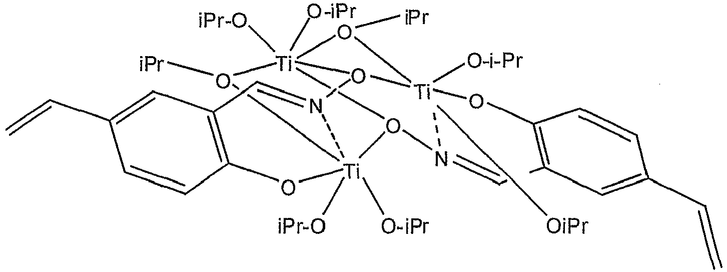 avec du divinylbenzène ou du triméthylolpropanetriacrylate.

15. Matériau polymérique dopé par au moins un élément métallique susceptible d'être obtenu par un procédé tel que défini selon l'une quelconque des définitions 6 à 14.

16. Utilisation d'un matériau tel que défini selon la revendication 15, en tant qu'élément de cible laser.

## Patentansprüche

1. Koordinationskomplex wenigstens eines metallischen Elements, ausgewählt aus Titan, Zirkon, Hafnium, Vanadium, Niob, Tantal mit wenigstens einem Monomer, das der nachfolgenden Formel (I) entspricht: 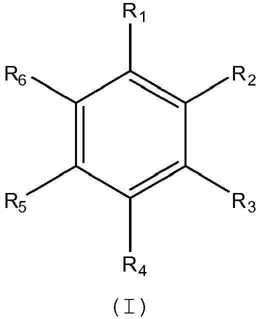 wobei:
- R₁ eine Ethylengruppe ist;
- R₂, R₃, R₄, R₅ und R₆ unabhängig voneinander ein Wasserstoffatom repräsentieren, eine Gruppe -OH, eine Amingruppe, eine Gruppe -CHO, eine Oximgruppe, eine Hydrazongruppe, eine Carboxylgruppe -COOH, ein Halogenatom, eine Trialkylsilangruppe und deren eventuelle Salze, unter der Voraussetzung, dass wenigstens eine der Gruppen R₂ bis R₆ eine Gruppe -OH repräsentiert, und wenigstens eine der Gruppen R₂ bis R₆ eine Oximgruppe repräsentiert ;
wobei das metallische Element in Form eines metallischen Alcoxyds ist.

2. Komplex nach Anspruch 1, wobei das Monomer wenigstens eine Gruppe -OH umfasst und wenigstens eine Oximgruppe, die zueinander in Orthoposition an ein und demselben aromatischen Ring angeordnet sind.

3. Komplex nach einem der vorhergehenden Ansprüche, wobei das Monomer der nachfolgenden Formel (II) entspricht:

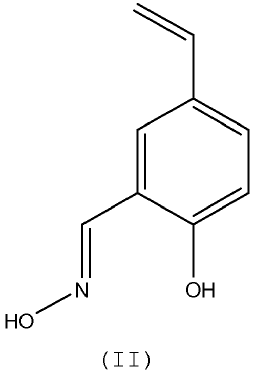

4. Komplex nach einem der vorhergehenden Ansprüche, wobei das metallische Element Titan ist.

5. Komplex nach einem der vorhergehenden Ansprüche, der einer der nachfolgenden Formeln (III) und (IV) entspricht: 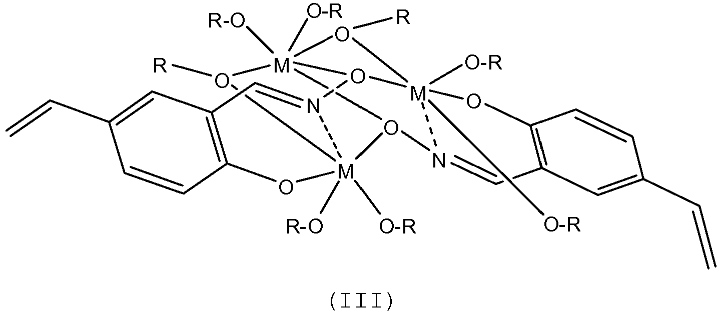 wobei M ein metallisches Element repräsentiert, und R eine Alkylgruppe repräsentiert; 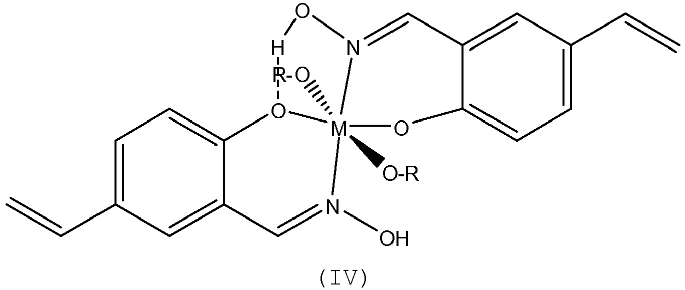 wobei M ein metallisches Element repräsentiert, und R eine Alkylgruppe repräsentiert.

6. Verfahren zur Herstellung eines Polymermaterials, das mit wenigstens einem metallischen Element dotiert ist, umfassend einen Schritt der Polymerisation wenigstens eines Koordinationskomplexes wie in einem der Ansprüche 1 bis 5 definiert.

7. Verfahren nach Anspruch 6, wobei der Schritt der Polymerisation in Anwesenheit eines oder mehrerer Komonomere durchgeführt wird.

8. Verfahren nach Anspruch 7, wobei das oder die Komonomere ausgewählt sind aus den Stryrolmonomeren und den Acrylatmonomeren.

9. Verfahren nach Anspruch 7 oder 8, wobei das oder die Komonomere wenigstens zwei Ethylengruppen umfassen.

10. Verfahren nach Anspruch 8 oder 9, wobei das oder die Komonomere einer der nachfolgenden Formeln (V) oder (VI) entsprechen: 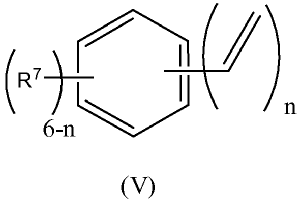 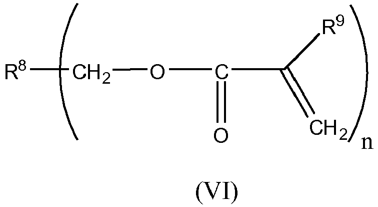 wobei die (6-n) R⁷, die identisch oder verschieden sind, ein Wasserstoffatom repräsentieren, eine Alkylgruppe, eine Arylgruppe, eine Gruppe -O-Aryl, eine Gruppe -O-Alkyl, eine Acylgruppe, eine Alkylarylgruppe, ein Halogenatom, wobei die Alkyl-, Aryl-, Alkylaryl-, -O-Aryl-, -O-Alkyl-Gruppen gegebenenfalls perfluoriert sind, wobei R⁸ eine Alkylgruppe repräsentiert, wobei R⁹ H oder eine Alkylgruppe repräsentiert, und n eine ganze Zahl ist, die von 1 bis 3 geht.

11. Verfahren nach Anspruch 10, wobei das Komonomer Divinylbenzol ist.

12. Verfahren nach Anspruch 10, wobei das Komonomer Trimethylolpropan-Triacrylat ist.

13. Verfahren nach einem der Ansprüche 6 bis 12, umfassend nach dem Schritt der Polymerisation einen Schritt der superkritischen CO₂-Trocknung.

14. Verfahren nach einem der Ansprüche 6 bis 13, wobei der Schritt der Polymerisation aus der Kopolymerisation eines Koordinationskomplexes der nachfolgenden Formel: 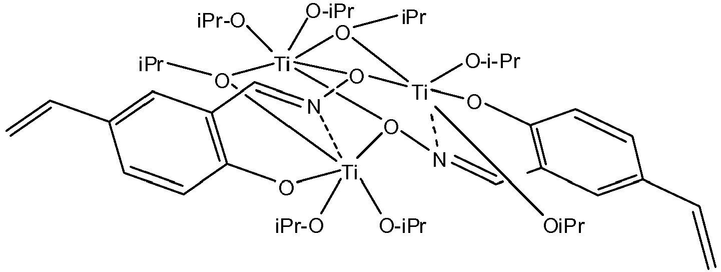 mit Divinylbenzol oder Trimethylolpropan-Triacrylat besteht.

15. Polymermaterial, das mit wenigstens einem metallischen Element dotiert ist, das durch ein Verfahren wie in einem der Ansprüche 6 bis 14 definiert erhältlich ist.

16. Verwendung eines Materials wie in Anspruch 15 definiert als Laserzielelement.

## Claims

1. A coordination complex of at least one metal element chosen from titanium, zirconium, hafnium, vanadium, niobium, tantale with at least one monomer fitting the following formula (I): 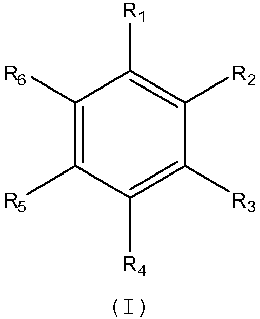 wherein:
- R₁ is an ethylenic group;
- R₂, R₃, R₄, R₅ and R₆ represent independently of each other, a hydrogen atom, a -OH group, an amine group, a -CHO group, an oxime group, a hydrazone group, a carboxyl group -COOH, a halogen atom, a trialkylsilane group, and optional salts thereof, provided that at least one of the groups R₂ to R₆ represents a -OH group and at least one of the groups R₂ to R₆ represents an oxime group;
said metal element being in the form of a metal alkoxide.

2. The complex according to claim 1, wherein the monomer comprises at least one -OH group and at least one oxime group located in an ortho position relatively to each other on a same aromatic ring.

3. The complex according to any of the preceding claims, wherein the monomer fits the following formula (II):

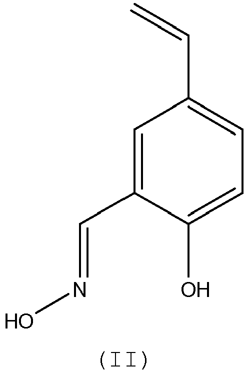

4. The complex according to any of the preceding claims, wherein the metal element is titanium.

5. The complex according to any of the preceding claims, fitting one of the following formulas (III) and (IV): 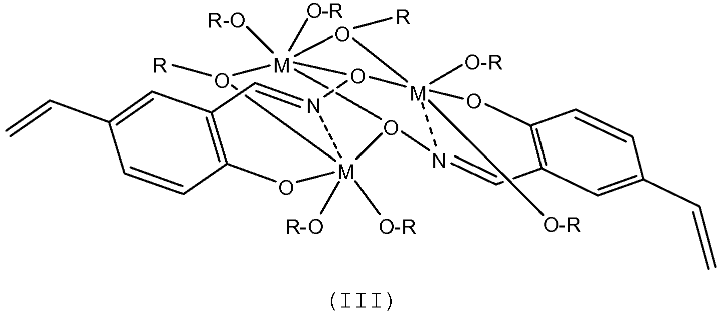 with M representing a metal element and R representing an alkyl group; 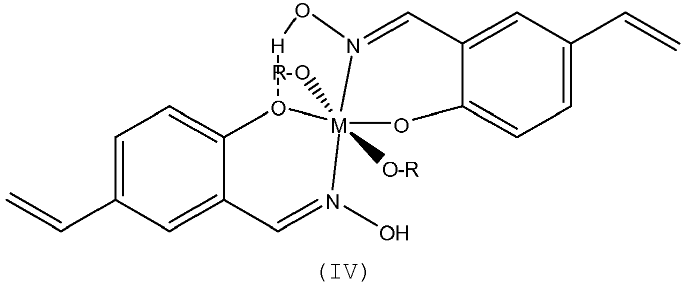 with M representing a metal element and R representing an alkyl group.

6. A method for preparing a polymeric material doped with at least one metal element comprising a step for polymerizing at least one coordination complex as defined according to any of claims 1 to 5.

7. The method according to claim 6, wherein the polymerization step is carried out in the presence of one or several comonomers.

8. The method according to claim 7, wherein the comonomer(s) is(are) selected from styrenic monomers and acrylate monomers.

9. The method according to claim 7 or 8, wherein the comonomer(s) comprise(s) at least two ethylenic groups.

10. The method according to claim 8 or 9, wherein the comonomer(s) fit(s) one of the following formulae (V) or (VI): 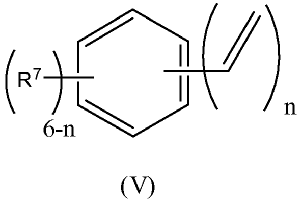 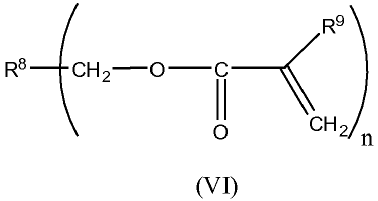 wherein the (6-n) R⁷ groups, either identical or different, represent a hydrogen atom, an alkyl group, an aryl group, an -O-aryl group, an -O-alkyl group, an acyl group, an alkylaryl group, a halogen atom, said alkyl, aryl, alkylaryl, -O-aryl, -O-alkyl groups being optionally perfluorinated, R⁸ represents an alkyl group, R⁹ represents H or an alkyl group and n is an integer ranging from 1 to 3.

11. The method according to claim 10, wherein the comonomer is divinylbenzene.

12. The method according to claim 10, wherein the comonomer is trimethylolpropane triacrylate.

13. The method according to any of claims claim 6 to 12, comprising, after the polymerization step, a step for supercritical drying with CO₂.

14. The method according to any of claims 6 to 13, wherein the polymerization step consists in the copolymerization of a coordination complex of the following formula: 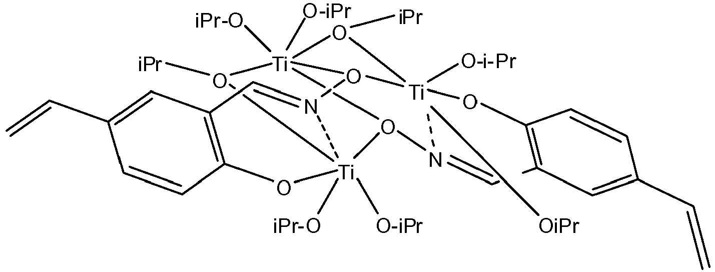 with divinylbenzene or trimethylolpropane triacrylate.

15. A polymeric material doped with at least one metal element which may be obtained by a method as defined according to any of the definitions 6 to 14.

16. The use of the material as defined in claim 15, as a laser target element.
